# EUROPEAN PATENT APPLICATION

(11) **EP 2 388 338 A1**
(43) Date of publication of application: **23.11.2011**
(21) Application number: 10161687.8
(22) Date of filing: 30.04.2010
(51) Int. Cl.: C12Q 1/68

(54) **Method for determination of and medicament for influencing the activity of the immune system**

(71) Applicant: Medizinische Hochschule Hannover, 30625 Hannover (DE)
(72) Inventor: Ferreira de Figueiredo, Constanca, 30171 Hannover (DE); Blasczyk, Rainer, 30938 Burgwedel (DE); Gras, Christiane, 30171 Hannover (DE)
(74) Representative: Taruttis, Stefan Georg

(57) **Abstract**

The present invention provides a method for the determination of the state of the immune system, e.g. the state of activity and activation, respectively, of the immune system, using the determination of presence of a blood or peripheral mononuclear cell or plasma marker. The marker can be determined directly, e.g. by determination of its titre using a specific antibody against the marker, or indirectly, e.g. by the determination of the mRNA encoding the marker.

Further, the invention relates to the use of the protein corresponding to the marker analysed in the analytical method, as a medicament, especially for influencing, e.g. for activating the immune system, more specifically for activating the cytotoxicity ofNK cells as well as for activating the cytotoxicity and the proliferation of T-cells.

SEQUENCE LISTING

<110>  Medizinische Hochschule Hannover

<120>  Method for the determination of and medicament for influencing
       the activity of the immune system

<130>  M1029EP

<160>  11

<170>  PatentIn version 3.5

<210>  1
<211>  17
<212>  DNA
<213>  Artificial Sequence

<220>
<223>  /note="SEQ ID NO 1: synthetic primer"

<400>  1
caacggcggc cactatg                                                    17


<210>  2
<211>  15
<212>  DNA
<213>  Artificial Sequence

<220>
<223>  /note="SEQ ID NO 2: synthetic primer"

<400>  2
ccaggcgggc tttgc                                                      15


<210>  3
<211>  18
<212>  DNA
<213>  Artificial Sequence

<220>
<223>  /note="SEQ ID NO 3: synthetic probe"

<400>  3
caacgattcc gatacaca                                                   18


<210>  4
<211>  20
<212>  DNA
<213>  Artificial Sequence

<220>
<223>  /note="SEQ ID NO 4: synthetic primer"

<400>  4
ttcagcaatg gagctttcca                                                 20


<210>  5
<211>  18
<212>  DNA
<213>  Artificial Sequence

<220>
<223>  /note="SEQ ID NO 5: synthetic primer"

<400>  5
caaccccagc cgttgaac                                                    18


<210>  6
<211>  19
<212>  DNA
<213>  Artificial Sequence

<220>
<223>  /note="SEQ ID NO 6: synthetic probe"

<400>  6
aagcagcaac aactatata                                                   19


<210>  7
<211>  18
<212>  DNA
<213>  Artificial Sequence

<220>
<223>  /note="SEQ ID NO 7: synthetic primer"

<400>  7
atgatgatat cgccgcgc                                                    18


<210>  8
<211>  16
<212>  DNA
<213>  Artificial Sequence

<220>
<223>  /note="SEQ ID NO 8: synthetic primer"

<400>  8
gccttgcaca tgccgg                                                      16


<210>  9
<211>  15
<212>  DNA
<213>  Artificial Sequence

<220>
<223>  /note="SEQ ID NO 9: synthetic probe"

<400>  9
cgtcgtcgac aacgg                                                       15


<210>  10
<211>  11825
<212>  DNA
<213>  Human semaphorin 5A

<400>  10
   gctctccgcc ccgcgctctc cgcgcccgct cgccccgcgc cgatctactt gccgggaagg      60
   cggcgccggt ggcggctgct ctccctgagc ccgctcccga gcgctgcttt cccgccgcgg     120
   gtgggcttcg cagcctcagg ccagccgcgg cccttggccc gctgcagccc cggccctcca     180
   ccttccccgt gcaggggcgg cccggccagt gtcgctcatc ccgggacgct cccttctccc     240
   acccaggact gccccgcgga gctggcttgg acacccaact ttgccacctc gagggtcgtc     300
   tctgctgggc gcgaacctgc ccacccaccg gttggccgcg cgctcgggga ccgtgctcgt     360
   ggcccccaag ccggtgcccc cattctggaa ctcagcgagt agggggcggc tctggggaag 420
   tggcaggggg cggctgcagc tgctgcctcc acttccctag ccaggtgctg aagaggatcc 480
   tcggagccgc tctggccccc aggcgctgga tgactggcac cagcgctcct cgcacctgtg 540
   ttggtgtgtg agacttgggc tggagtgccc acgtggctgt ggagtcagtg tgattcatga 600
   ttgaggaaac gcgtcctcca tcctctctct ccttggcact ttccacacat gaggagaaga 660
   agagcttctg tttagaagac acgtgcccag agtcagaggc cccttgccca ccatgaaggg 720
   aacctgtgtt atagcatggc tgttctcaag cctggggctg tggagactcg cccacccaga 780
   ggcccagggt acgactcagt gccagagaac cgagcatcca gtcatctcct ataaagaaat 840
   tggcccctgg ttacgggagt tcagagcgaa gaatgctgtg gatttctcgc agttaacatt 900
   tgacccagga cagaaagaac ttgttgtagg agcaagaaac tacctcttca ggttacagct 960
   tgaggatctg tctcttatcc aggctgtgga atgggagtgt gatgaagcta ccaaaaaggc 1020
   ctgttacagc aaaggcaaat caaaggagga atgtcagaac tacatccggg tgcttctggt 1080
   gggtggcgac cggttattca cctgtgggac caatgcattc acgcctgtct gcaccaaccg 1140
   ctcgttgagc aacctgactg agatccatga tcagatcagt ggcatggccc gctgtcccta 1200
   cagtccccag cacaattcca cagcgctcct cacagctggt ggggagctct atgctgctac 1260
   agccatggat tttccaggac gtgatcctgc catttaccga agcctaggca ttttacctcc 1320
   tctccgcacg gcgcagtaca actccaaatg gctcaatgag ccaaactttg tgtcatctta 1380
   tgacatcgga aattttacct acttcttttt ccgagaaaat gcagtagagc atgactgtgg 1440
   gaaaacagtg ttctccagag ctgcccgggt gtgcaagaac gatattggtg ggcgcttcct 1500
   gctggaagac acctggacca cattcatgaa ggctcgcctg aactgctccc gtcctgggga 1560
   agtccccttt tactacaacg aattgcagag tactttcttc ctgcctgagc tggatttgat 1620
   ctatggcatc tttaccacca atgtgaacag cattgcggcc tcagctgtgt gcgtcttcaa 1680
   cctgagcgcc atcgcgcagg ccttctctgg gcccttcaag taccaagaaa actcgcgctc 1740
   ggcctggcta ccgtatccca acccaaaccc ccacttccag tgtggcaccg tggaccaggg 1800
   cctgtacgtg aacctgaccg agagaaatct gcaggatgct cagaagttca ttctgatgca 1860
   tgaggtggta cagccagtga ccacagtgcc ctccttcatg gaggacaata gccgcttttc 1920
   ccacgtggca gtcgacgtgg tgcagggcag agaagcgctc gtccacatca tctatttggc 1980
   cacagattac ggaaccatta agaaagtgcg ggtacccctg aatcagacct caagcagctg 2040
   tttgctggaa gagattgagc tcttccctga gaggcggagg gagcccatca ggagcctgca 2100
   gatcctgcac agccagagtg tcctgttcgt gggcctgcgg gagcacgtgg tcaagatccc 2160
   cctgaagagg tgccagttct accgcacacg cagcacctgc attgggcccc aggaccctta 2220
   ctgtggctgg gatgtggtaa tgaagaaatg cacaagcctg gaggagagcc tgagcatgac 2280
   gcagtgggaa cagagcatct ctgcgtgtcc gaccaggaat ctcaccgtgg atgggcactt 2340
   tggtgtgtgg tctccgtgga cgccttgcac gcacacagat ggcagcgccg tgggatcctg 2400
   cctctgtcga acccgctcct gcgacagccc ggccccgcag tgtggtggct ggcagtgcga 2460
   gggccctggc atggagatcg ccaactgttc caggaacgga ggctggactc cctggacctc 2520
   gtggtctccc tgcagcacta cctgtgggat cggcttccag gtgcggcagc gctcctgcag 2580
   caaccccact cccaggcacg ggggccgggt gtgcgtggga cagaaccgcg aggaaagata 2640
   ctgcaatgaa catttgctat gtcccccaca catgttctgg acaggctggg gtccttggga 2700
   acggtgcaca gcccaatgcg ggggtggcat tcaagctcgc cgcaggatct gtgagaatgg 2760
   gcctgactgt gcaggctgca atgtggagta ccagtcttgc aacaccaacc cgtgtcctga 2820
   gctgaagaag accacgccct ggacaccctg gacacctgtc aacatctctg acaacggcgg 2880
   ccactatgag caacgattcc gatacacatg caaagcccgc ctggctgatc cgaatttgct 2940
   ggaagtggga agacagagaa tcgaaatgcg gtactgttct agcgacggca ccagtggctg 3000
   ctccacagat gggctttctg gggatttcct gcgtgctggg agatactctg cccacacggt 3060
   caacggggct tggtcagcct ggacgtcgtg gtcacagtgc agccgtgact gcagcagggg 3120
   cattcggaac cggaagcgtg tttgcaacaa ccccgaaccc aagtatgggg gaatgccttg 3180
   ccttggccca tctctggaat accaggaatg caacattttg ccctgcccag tggatggcgt 3240
   gtggtcttgc tggtccccct ggacaaaatg ttcagcaaca tgcggcggtg gacactatat 3300
   gaggacccgc tcttgctcca atccagcccc ggcctatgga ggggacatct gcctggggct 3360
   gcacacagaa gaggcactct gcaacacgca gccctgccca gagagctggt cggagtggtc 3420
   ggactggtct gagtgtgaag cctctggcgt ccaagtccgc gcccgccagt gcatcctcct 3480
   gttccccatg ggcagccagt gctccgggaa caccacggag agccggccgt gtgtgtttga 3540
   ctctaatttc atcccagaag tatctgtggc aagatccagt agcgtagaag agaaaaggtg 3600
   tggagagttc aacatgttcc acatgatcgc cgtggggctg agcagctcca tcctcggctg 3660
   cctcctcacc ctgctcgtct atacttactg ccagcggtac cagcagcaat cccacgatgc 3720
   gactgtcatc caccccgtct cacctgcccc ccttaatacc agcataacca accacatcaa 3780
   caaactggac aagtacgact cggtggaggc catcaaggca tttaacaaaa acaacttgat 3840
   cctagaggaa agaaacaaat acttcaaccc acatctcact gggaagacct attctaatgc 3900
   ctactttaca gatctcaata attatgatga gtactaacag ctttcatgtt tttggcttct 3960
   tgtaaatccc cagttcctca aggcctgtgc cccatgactg cccatgtttc tgaggcttca 4020
   gagtcgaagt ttggatacat ttcaagtgca tttcaagcca ccagagtgtc ccattgctgc 4080
   caaaaataca cgtctttaaa agcaacaaaa attgaaataa gacatcgtga aaatcttgac 4140
   cattgttgaa tgagccaggg tgtgaagttt ttaattgtgt tcatcctatt tttctgacaa 4200
   gtccattggt ttgttttttg agcattattt tataaatgtg ccacccacat tggaaggagt 4260
   ctttctttag aactttggag tgtaaatctt catgatgttg taattcaaga aaataggcac 4320
   tttctctgaa agacctgctc cttccacaag aagtgcatag gtccataata tttcataaaa 4380
   tgaagaaaaa gaatgtggcc aaacaattat tcaccatgga ttgcccaact ttccaaatct 4440
   ggataaagct gtgggattct tggaagcagc ttgagtgttt tcatcttgcc tgggaagccc 4500
   aggaattcca cctggtccac accggcagaa gttacagtag actgtgaggc accccacctt 4560
   gctcctgatg cagtttctgt gccattgctg gtgctggtgg aggcagcgga gcagaggctc 4620
   aggcacaatg aagcgtggat gtgttctgca ggttgctgca aaactcacct tattctgact 4680
   tttggatttc atggcattcc aggaagctcc ttgccatgct gttggcctgg aagtccacct 4740
   gtctggtcca tagtgacgtc ctgaagagcc agtctgtaaa ataaccaacc acttacttag 4800
   cgtttggata gactccatgc cttctctctc cctgcaaaga aaaatcttga acatttatga 4860
   tgtcaattag tgaaagtatt gaaaatacta aattataact aaaagcaact ttttatgtta 4920
   ttgaaaatat tgaaagaact gatattaatg ataatcattt tatttttcat ctctctgata 4980
   tacccaatgt gtggcaatca gcccctacca cgagcattaa tgccatgtaa agctggcttt 5040
   ctggagtctg ccaggtccac acgaagtgct acccccagtt tctgctgtta cttgctgcct 5100
   ccaggccagg ggagcaggag atgctcagct ctggtgcctt ttcttttatt tcagtgctgc 5160
   cttcccaccc acccagtcca ttcaccctca cctctccctg catggaggca acagcatttc 5220
   aagatgtacc ttgggaagtc aggatagctg gaagtaaggg tttttgggat ccctgtggtc 5280
   tcttcactga tcatcgataa gcatttaaga gtgtgctaac accattcaca gaggtccctg 5340
   gaaaaaaata tataattttc tcacaaacaa ccaatacata acctatggca gttggttgaa 5400
   taattattga aaaaaagaac acgactgaaa aagttcatct gatgcctctg agcatgtgat 5460
   aaagtcctgg gtagacacgg aaacgtgttc ttacaaatga accttcggat ctatgaaaga 5520
   aaatatagta gggggactaa ggaacataga attttattag catatgtgat tttaccctta 5580
   tctttgtttc taatttaaag aaacaatttc agaaagtgtt agaagaattc tatgtttaat 5640
   aatgaatatt gttgagttca aaatattctc atatacccag atttacagag atgacacagt 5700
   attgaaatgg caggtgggct ctgtaagtta ttttggatta atgacattca gggtctttgc 5760
   agtggggact tcatgttgcc tctcactcat tcctcatatg tcagagtctt ctgtctatat 5820
   gcacatgctc aaagtccata ctcagtaggg gaaatctagc cggatggttc tccaattgtt 5880
   cctcggctgg attttcatca tcagatattt caactcatct catcagtttc ttgtaagaaa 5940
   aaaacatttt cactttgtag cacattttag ttatttttga gtcttcttcc ctaatagttt 6000
   gtaagcttaa agggacattt tattttccct ggtaaagagg attctaatat ttcaagaatg 6060
   tttcactgga attgatggga aatggtttct aaatggcatc gaagctggtc tttaatgagt 6120
   cttttgcaat gacttgggaa aaacaccctc cctcactaca ggatcttcat gttgttaata 6180
   atataaacaa acctttgaaa ttagcattgc aaaagaatcc catttttgtt tcgtaccaca 6240
   ctgttcaaag gaaaattgtt tatctctctt cctttctctc tctctcttat ttgacagaat 6300
   agcaagtgtt tagataattt cagatatatt ctaagtattt taccagctgt ggaataagtt 6360
   gtgtttgtcc atcactgtgt agctaccgtt cacatgttct ggctctgtac tccacgctta 6420
   tcgcgtgaac cctcacatgt tggactttca cagtgtgatc tctcacctgt cttggaaact 6480
   ccttcataaa gccgctcttc cttaggcctg ggctgtttgg aagtcctggt gaaactttgc 6540
   ggttcacatt ttaaattcct gaatgacctt ttcattctct ctttctttat ctttgttttt 6600
   gtccttcatt ccctcctttt gttcttcctt cctgccttct tttcttcttt cctttcttct 6660
   ttctttaatt cttcctttct tcctattttc ttggtacaag tggaacaaaa accaacacat 6720
   agttttaagc tacacctttc tgcacctgat gtaaaatgac attaatcact atttaacttc 6780
   taaaattatt tctgaataca gttggagata ggctggtttt catgaggaag ctggcttggc 6840
   tttagtctta catttaaatt ctttgaaagt ggctcccagt gctattcagg gtcctttctt 6900
   gaggccagac tcatcaccat ctactattga ttttacagtg cactgacagt ttacaggaag 6960
   gagaagaaca gaatttctgc acactacaca acatgtgggc ttcctgtagc tccagggaca 7020
   tgtagctttg gtgaaactgg ggttttgtaa cctctgaatg atatggactt agtgaattct 7080
   aaggaactca gggggcacgt ggtcaggctc caccgcacag aagagccaca gtctccagac 7140
   tcatggcgtt ccctccagaa ctccccattc ccctctgagc atatttctat gctgctgctt 7200
   cttgtgactt acatgaggca tctcagcttc ttcatgtttg tagggatggt tccctaagcc 7260
   tgttcaagta ggctctcact agagttacca ttcatatttg agaagaaaag aacatttaat 7320
   aaatgtatgt gtggggctga ggttctaaag gaattgaaaa gagacgataa acattgaatg 7380
   agggtgaggg catccctgct ggggagaaac ctctgtccct aggaagagtc cgttcatgtc 7440
   atgtggtttg ggatgaacca ggggtctggc cccatcgggt cacaggtgat ggcaaataga 7500
   aaagaagcaa atggaggaaa tagtcggata agtaggtgac gtgaaaggca ggacctggtc 7560
   accccagcaa gtgctatgga cagttcccgg aaacggttgc ccacttcaca ggtccatggg 7620
   tctgaccctt ggactctgcc aggatcaact gcccagagtg ccagagtttt agccaaaggt 7680
   gtacttactt ccttatttat ctgcaaaagg atggaaactg tgggagtcaa agcctatttt 7740
   gctgagtgtt cccactggag tctctggtag aattagcagg tcatgctgtc aaaatcatgg 7800
   acaaaggctg ggtgcagtgg ctcatgccta taatcccagc actttgggag gccaaggtgg 7860
   gcggatcacc tgagctcagg agtttgagac cagcctgggc aacatgggga aactccatct 7920
   ctacaaaata tacaaaatat tagccagcca tcgtggtgcg tgcctgtggt cccagtttct 7980
   tgggaggctg aggcgggagt atcatttgag ccaggaggtt gaggttgcag tgagctgaga 8040
   tcacatcact gcactccatc ctgggtgaca gagagagacc ctgtctcaaa aaaaaaaaaa 8100
   aagaacaaag tcttagcggg gggtctctat gtccccaagt ggctctctaa ggggatccat 8160
   tagcgtataa ttgaatatca aagggaattt taccttaaac atatatcaag tctatactca 8220
   gctaatcgtt ttgtctaaac acagccttgt tagcatttga atttacaaat attcatttgc 8280
   ttatttgcag tgttcctttt gcttttagaa taaatcataa atatttcccc aatactgtca 8340
   agatacttct tcaactttgt ccctgaacca tcagctttct ttagggtccg ggtatccttt 8400
   acacttcaat ggggtttctt tgtgtgtgtc attgggggta actccattga cctcagcatt 8460
   tctatctcaa gagatggctc tggaacacag aaccagaggc aacaggtact aatggttgat 8520
   aaaattaaca gcgactgcat aaccattttc cacatggcag ggctgtgctt aagccccaca 8580
   cctccaataa tcaatttcta tgacggtact aataacctta actatatgca aattcttcac 8640
   cacttatgat atggtacaaa ctggagttta cagatggcct tggccttgcc tagcccattt 8700
   gttcatccta acttcttcat cacttcctga tgcacttggg gaagcatgtt gacacaacgt 8760
   taccaatatt tgattaccta tggttatctg caattaccta cgcttatctt atcagaccct 8820
   agatgccatt tgagcccctt acttctttgg gcagtgggtg aaatgggaga acatagagaa 8880
   atgttcactt tgacctaaga aacgcataaa agggaatgcc atgcacaatg taagataaaa 8940
   tgtttgggga caggggatca tcgtgaaatt tatttctgct attctgccct attgaggtca 9000
   ttaattataa tggatttaaa gatttttttt taaactctac aaacactaag ccaaggaatt 9060
   caggttatct gctatgttaa cctaataaag caaaggaaaa taagttgctt taatccttgc 9120
   ttgttaacct aataaagcaa aggaaaatat gttgctttaa tccttgcttc ttttctcttt 9180
   tggtaataga aaaggtaaat aagatgtaat gttgaactgc tggtgatgat ttctaaaacg 9240
   actctttaaa tactcctttc ctatggactt atattaaaga tgcattgtac acattgtttt 9300
   agcagataga tcaaagatat actcagtggt catctgttgc attatcagaa atttgataca 9360
   tgttacagaa gcatgaggaa gggaatccta tccacttttg aaaatagagt tcacattgca 9420
   agcgacatag ggcattatca aaaccaattc agagcctggc acaaaatgta tacttcctag 9480
   tgaaagctgt ctcaaaacac agcctttttg atggatcata ttagctcata tctgccatag 9540
   aaaggcaatc aagcttactc cctgcaatcg gagtcaatat caattcttgc tcaagcaaac 9600
   atgttttaca gtgttggcgc aatggcagga actagtcaag ggcactttca gatctggaaa 9660
   gaggaaagag gaggtagaaa atcactcttt tgtgtttctt aaactgtcaa tcaagcaaga 9720
   aaatttattc caccccttca tatttgctct aaaatctggg gccagtagtt gaaatgtgat 9780
   cttgttcttt ctcagccttt tggcaattta atgccaaata ttccaaatgt tagatgaatg 9840
   gcagtaataa aatgattcaa atgtttgata aatacaagct gagagcaaaa tctggactct 9900
   gaaaaatcgg aactatttta tcatgttgct aaaatgagag catcattttc ttccctctct 9960
   gtaagtgcgg tagtttaatt tcctagaaaa agttgctagt gccttgttta agatgatcat 10020
   ttatcatttc atgtggatat tattggtcta attagaagga gaagattgat tggatttact 10080
   ttaaagaaaa ttattctcct atgtctcttt gaactcagga atagaaaatg accaagaagc 10140
   acatcatctt cagatgtgat ttttgccctc actggggatg tagaaccaga ataggtacaa 10200
   catatgctca gaagagaggt gacttcagta ttagccttct agaaactaac atcactttaa 10260
   tttttacatg ttccaaaaaa aatcatagtg attgttctgt aataagaatg ttaacgtgat 10320
   gcctatagaa gttagaggtt tttgtttctg tttctcttca ggcaagtcca tagatagagg 10380
   aaaatcagac acaggctggt gacaactggt atagatacag tgagcatgga atgagaacca 10440
   cctacaacat tgcctgatta tttttaagtt tatatcaaaa ctgcttcaca agttcacaaa 10500
   agttgtcact cttaatttgg gttaagaaaa gtaatagtat tatctctgct gaagttgatt 10560
   gtttttctct tttaccatga ctgttggcca aaagcaagct gaacctctgc actttgaaac 10620
   aaagcacagt atgaatcttt aacccaagca cttagtttca ccactgagat tctacatcat 10680
   ctgcaagaaa atgcacaaac ttttggtcca atgttggatt cttcaaaagt gcatagcaaa 10740
   acttttgtgc tatgccgctt gatgtaactt ttaaagatgt tatcaaatgt taaatgcctc 10800
   attctgcgtt aatttgctgt tggctgtttt gtgaacacaa aataaacttg agattttttt 10860
   tttttttttt ttttgtgaaa ctcctccaaa gccaatttca gcccttggta agcttgcaga 10920
   cagttactga tcttaaattt aattttaaaa gacaatgtat cttaattata atttagcttt 10980
   ttaaaacaat gagatagctt tacatttccc ctttgtttga atgagaaaat ggatcttggg 11040
   ttgctatgct agaacacttg tagattgctg ggtcctttgt aagggggcca tggacacacc 11100
   acactttctt tcaatcctta catttgaagc attgatattc ttcaaaacct tcttgttaca 11160
   tgtgcgcaat agaaatttct aatgttcatg acttttatct ttcctgtcca tcaattcact 11220
   ggttgtaaat gcttcctgag agctgtctag gtctgtatcc cagattgttg cttaatgaca 11280
   tctgacagat gcattgtttt ctgaaatcag cttaagacac caattgtggc aactggaaac 11340
   tcattacctg ctgcattgga tcaactatgg aagttggagc aggggtgggc ggaggtcacc 11400
   taaccaatca atggaaggca actcacacct gctccaagcc tcagctttga gaaacaaaca 11460
   cgtttataag aaaaaatata tagctattat tacagaagtg aatatgttgt gctctcttac 11520
   tgctcttggt gcattgacag tttctgtatc tcaaccctat tcatctttat gaaaaagcat 11580
   tctgaagatc tatcctcagc actgctgagt gtgcagtcac actttcctac caaccccctt 11640
   cttaccatct ctagctgcca tttgtggggg gaacaaaaca agggaatcct gattgtgtac 11700
   agtataaatt gtattatatt ttttgtactt atgttttatg taaatagttt gtctcattca 11760
   attgtatgtg agcattgaaa taaatcctac catttagggg acaatttaaa aaaaaaaaaa 11820
   aaaaa 11825

<210> 11
<211> 1074
<212> PRT
<213> Human semaphorin 5A

<400> 11

Met Lys Gly Thr Cys Val Ile Ala Trp Leu Phe Ser Ser Leu Gly Leu
1               5                   10                  15

Trp Arg Leu Ala His Pro Glu Ala Gln Gly Thr Thr Gln Cys Gln Arg
            20                  25                  30

Thr Glu His Pro Val Ile Ser Tyr Lys Glu Ile Gly Pro Trp Leu Arg
        35                  40                  45

Glu Phe Arg Ala Lys Asn Ala Val Asp Phe Ser Gln Leu Thr Phe Asp
    50                  55                  60

Pro Gly Gln Lys Glu Leu Val Val Gly Ala Arg Asn Tyr Leu Phe Arg
65                  70                  75                  80

Leu Gln Leu Glu Asp Leu Ser Leu Ile Gln Ala Val Glu Trp Glu Cys

                85                  90                  95

Asp Glu Ala Thr Lys Lys Ala Cys Tyr Ser Lys Gly Lys Ser Lys Glu
            100                 105                 110

Glu Cys Gln Asn Tyr Ile Arg Val Leu Leu Val Gly Gly Asp Arg Leu
        115                 120                 125

Phe Thr Cys Gly Thr Asn Ala Phe Thr Pro Val Cys Thr Asn Arg Ser
    130                 135                 140

Leu Ser Asn Leu Thr Glu Ile His Asp Gln Ile Ser Gly Met Ala Arg
145                 150                 155                 160

Cys Pro Tyr Ser Pro Gln His Asn Ser Thr Ala Leu Leu Thr Ala Gly
                165                 170                 175

Gly Glu Leu Tyr Ala Ala Thr Ala Met Asp Phe Pro Gly Arg Asp Pro
            180                 185                 190

Ala Ile Tyr Arg Ser Leu Gly Ile Leu Pro Pro Leu Arg Thr Ala Gln
        195                 200                 205

Tyr Asn Ser Lys Trp Leu Asn Glu Pro Asn Phe Val Ser Ser Tyr Asp
    210                 215                 220

Ile Gly Asn Phe Thr Tyr Phe Phe Phe Arg Glu Asn Ala Val Glu His
225                 230                 235                 240

Asp Cys Gly Lys Thr Val Phe Ser Arg Ala Ala Arg Val Cys Lys Asn
                245                 250                 255

Asp Ile Gly Gly Arg Phe Leu Leu Glu Asp Thr Trp Thr Thr Phe Met
            260                 265                 270

Lys Ala Arg Leu Asn Cys Ser Arg Pro Gly Glu Val Pro Phe Tyr Tyr
        275                 280                 285

Asn Glu Leu Gln Ser Thr Phe Phe Leu Pro Glu Leu Asp Leu Ile Tyr
    290                 295                 300

Gly Ile Phe Thr Thr Asn Val Asn Ser Ile Ala Ala Ser Ala Val Cys
305                 310                 315                 320

Val Phe Asn Leu Ser Ala Ile Ala Gln Ala Phe Ser Gly Pro Phe Lys
                325                 330                 335

Tyr Gln Glu Asn Ser Arg Ser Ala Trp Leu Pro Tyr Pro Asn Pro Asn
            340                 345                 350

Pro His Phe Gln Cys Gly Thr Val Asp Gln Gly Leu Tyr Val Asn Leu
        355                 360                 365

Thr Glu Arg Asn Leu Gln Asp Ala Gln Lys Phe Ile Leu Met His Glu
    370                 375                 380

Val Val Gln Pro Val Thr Thr Val Pro Ser Phe Met Glu Asp Asn Ser
385                 390                 395                 400

Arg Phe Ser His Val Ala Val Asp Val Val Gln Gly Arg Glu Ala Leu
                405                 410                 415

Val His Ile Ile Tyr Leu Ala Thr Asp Tyr Gly Thr Ile Lys Lys Val
            420                 425                 430

Arg Val Pro Leu Asn Gln Thr Ser Ser Ser Cys Leu Leu Glu Glu Ile
        435                 440                 445

Glu Leu Phe Pro Glu Arg Arg Arg Glu Pro Ile Arg Ser Leu Gln Ile
    450                 455                 460

Leu His Ser Gln Ser Val Leu Phe Val Gly Leu Arg Glu His Val Val
465                 470                 475                 480

Lys Ile Pro Leu Lys Arg Cys Gln Phe Tyr Arg Thr Arg Ser Thr Cys
                485                 490                 495

Ile Gly Ala Gln Asp Pro Tyr Cys Gly Trp Asp Val Val Met Lys Lys
            500                 505                 510

Cys Thr Ser Leu Glu Glu Ser Leu Ser Met Thr Gln Trp Glu Gln Ser
        515                 520                 525

Ile Ser Ala Cys Pro Thr Arg Asn Leu Thr Val Asp Gly His Phe Gly
    530                 535                 540

Val Trp Ser Pro Trp Thr Pro Cys Thr His Thr Asp Gly Ser Ala Val
545                 550                 555                 560

Gly Ser Cys Leu Cys Arg Thr Arg Ser Cys Asp Ser Pro Ala Pro Gln
                565                 570                 575

Cys Gly Gly Trp Gln Cys Glu Gly Pro Gly Met Glu Ile Ala Asn Cys
            580                 585                 590

Ser Arg Asn Gly Gly Trp Thr Pro Trp Thr Ser Trp Ser Pro Cys Ser
        595                 600                 605

Thr Thr Cys Gly Ile Gly Phe Gln Val Arg Gln Arg Ser Cys Ser Asn
    610                 615                 620

Pro Thr Pro Arg His Gly Gly Arg Val Cys Val Gly Gln Asn Arg Glu
625                 630                 635                 640

Glu Arg Tyr Cys Asn Glu His Leu Leu Cys Pro Pro His Met Phe Trp
                645                 650                 655

Thr Gly Trp Gly Pro Trp Glu Arg Cys Thr Ala Gln Cys Gly Gly Gly
            660                 665                 670

Ile Gln Ala Arg Arg Arg Ile Cys Glu Asn Gly Pro Asp Cys Ala Gly
        675                 680                 685

Cys Asn Val Glu Tyr Gln Ser Cys Asn Thr Asn Pro Cys Pro Glu Leu
    690                 695                 700

Lys Lys Thr Thr Pro Trp Thr Pro Trp Thr Pro Val Asn Ile Ser Asp
705                 710                 715                 720

Asn Gly Gly His Tyr Glu Gln Arg Phe Arg Tyr Thr Cys Lys Ala Arg
                725                 730                 735

Leu Ala Asp Pro Asn Leu Leu Glu Val Gly Arg Gln Arg Ile Glu Met
            740                 745                 750

Arg Tyr Cys Ser Ser Asp Gly Thr Ser Gly Cys Ser Thr Asp Gly Leu
        755                 760                 765

Ser Gly Asp Phe Leu Arg Ala Gly Arg Tyr Ser Ala His Thr Val Asn
    770                 775                 780

Gly Ala Trp Ser Ala Trp Thr Ser Trp Ser Gln Cys Ser Arg Asp Cys
785                 790                 795                 800

Ser Arg Gly Ile Arg Asn Arg Lys Arg Val Cys Asn Asn Pro Glu Pro
                805                 810                 815

Lys Tyr Gly Gly Met Pro Cys Leu Gly Pro Ser Leu Glu Tyr Gln Glu
            820                 825                 830

Cys Asn Ile Leu Pro Cys Pro Val Asp Gly Val Trp Ser Cys Trp Ser
        835                 840                 845

Pro Trp Thr Lys Cys Ser Ala Thr Cys Gly Gly Gly His Tyr Met Arg
    850                 855                 860

Thr Arg Ser Cys Ser Asn Pro Ala Pro Ala Tyr Gly Gly Asp Ile Cys
865                 870                 875                 880

Leu Gly Leu His Thr Glu Glu Ala Leu Cys Asn Thr Gln Pro Cys Pro
                885                 890                 895

Glu Ser Trp Ser Glu Trp Ser Asp Trp Ser Glu Cys Glu Ala Ser Gly
            900                 905                 910

Val Gln Val Arg Ala Arg Gln Cys Ile Leu Leu Phe Pro Met Gly Ser
        915                 920                 925

Gln Cys Ser Gly Asn Thr Thr Glu Ser Arg Pro Cys Val Phe Asp Ser
    930                 935                 940

Asn Phe Ile Pro Glu Val Ser Val Ala Arg Ser Ser Ser Val Glu Glu
945                 950                 955                 960

Lys Arg Cys Gly Glu Phe Asn Met Phe His Met Ile Ala Val Gly Leu
                965                 970                 975

Ser Ser Ser Ile Leu Gly Cys Leu Leu Thr Leu Leu Val Tyr Thr Tyr
            980                 985                 990

Cys Gln Arg Tyr Gln Gln Gln Ser  His Asp Ala Thr Val  Ile His Pro
        995                 1000                1005

Val Ser  Pro Ala Pro Leu Asn  Thr Ser Ile Thr Asn  His Ile Asn
    1010                1015                1020

Lys Leu  Asp Lys Tyr Asp Ser  Val Glu Ala Ile Lys  Ala Phe Asn
    1025                1030                1035

Lys Asn  Asn Leu Ile Leu Glu  Glu Arg Asn Lys Tyr  Phe Asn Pro
    1040                1045                1050

His Leu  Thr Gly Lys Thr Tyr  Ser Asn Ala Tyr Phe  Thr Asp Leu
    1055                1060                1065

Asn Asn  Tyr Asp Glu Tyr
    1070

## Description

The present invention relates to a diagnostic method for the determination of the state of the immune system, e.g. the state of activity and activation, respectively, of the immune system, especially ofNK-cells and of T-cells, using the determination of presence of a blood or peripheral mononuclear cell or plasma marker. The marker can be determined directly, e.g. by determination of its titre using a specific antibody against the marker, or indirectly, e.g. by the determination of the mRNA encoding the marker.

Further, the invention relates to the use of the protein corresponding to the marker analysed in the analytical method, as a medicament, especially for influencing, e.g. for activating the immune system, more specifically for activating the cytotoxicity ofNK cells as well as for activating the cytotoxicity and the proliferation of T-cells.

The diagnostic method is especially suitable for use in the determination of the status of the immune system in samples obtained from patients having received a transplant, which especially is an allogeneic transplant of stem cells, e.g. of bone marrow stem cells in order to determine the state of the graft-versus-host (GvH) disease. Further, the diagnostic method is suitable for determination of the status of the immune system in a sample obtained from a patient having an autoimmune disease, or for monitoring the state of activity of the immune system in a sample obtained from a patient who is receiving medication influencing the activity of the immune system.

In a second aspect, the invention especially relates to the activation of the immune system by making use of the characteristics of the protein to activate the immune response, e.g. in an immuno-compromised patient. Therein, the invention relates to the compound for use as a medicament or for use in the manufacture of a medicament, especially for the treatment of a low activity of the immune system.

### State of the art

Generally, for diagnosis of the GvH disease, which is indicated e.g. by skin rashes, an organ biopsy is generally used.

Paczesny et al. (Science Translational Medicine 2, 13ra2 (2010)) describe the analysis of Elafin as a specific biomarker in plasma for the graft versus host disease.

### Objects of the invention

It is an object of the present invention to provide for an alternative diagnostic test method that can reliably determine the state of the immune system, e.g. a state of the immune response indicative of the GvH disease. A further object is to provide a compound for use in the activation of the immune system.

### General description of the invention

The invention achieves the above-mentioned objects by the features defined in the claims, and especially by providing an analytical or diagnostic method, and an analytical or diagnostic test kit suitable for performing the method, which analyses the concentration of Semaphorin 5A, either of Semaphorin 5A (SEMA5A) protein or peptide, and/or of Semaphorin 5A-specific mRNA. In this first embodiment of the invention, there is provided a method for determining the state of activity of the immune system by determining the concentration of Semaphorin 5A, either by determining the concentration of Semaphorin 5A protein, and/or by the determining the concentration of Semaphorin 5A-specific mRNA.

In the second embodiment, the present invention provides for Semaphorin 5A for use in the production of a pharmaceutical composition, and Semaphorin 5A for use in the treatment of a lack of immune activity, i.e. for increasing the activity of the immune system, especially for increasing the cytotoxic activity of NK cells, and for increasing the proliferation and the cytotoxic activity of T-cells, especially CD8⁺-T-cells. In greater detail, Semaphorin 5A is provided for use as a medicament, especially as an agent for stimulating and/or activating the immune system, which activation of the immune system preferably comprises or consists of activating NK cells, increasing the cytotoxicity of NK cells, and increasing the proliferation of T-cells, especially CD8⁺-T-cells. Preferably, Semaphorin 5A is for use as a medicament in combination with IL-2 and/or IL-15. Accordingly, this embodiment also relates to a medicament comprising Semaphorin 5A which is adapted or prepared for the medical treatment, especially for the activation of the immune system.

The embodiments of the invention are based on the observation that the titre of Semaphorin 5A and of Semaphorin 5A-mRNA is increased in patients being suspected of GvH disease or having clinically manifest GvH disease by a factor of about 20 in comparison to healthy patients. Further, it has been observed that the immune system can be activated by administration of Semaphorin 5A protein, as e.g. exemplified by the *in vitro* activation of the cytotoxic activity and increased cytotoxic activity ofNK cells by Semaphorin 5A, and as exemplified by the proliferation of CD8⁺ T-cells upon addition of the recombinantly produced Semaphorin 5A protein to the cultivation medium.

### Detailed description of the invention

The invention will now be described in greater detail with reference to the figures, wherein
- Figure 1 shows mRNA levels of Semaphorin 5A in PBMCs in allogeneic hematopoietic stem cell transplant recipients,
- Figure 2 shows mRNA levels of Semaphorin 3A in hematopoietic stem cell therapy patients,
- Figure 3 shows an SDS-PAGE of Semaphorin 5A expressed from transfected cells,
- Figure 4 shows the activation of NKp44⁺ cells upon stimulation by Semaphorin 5A,
- Figure 5 shows the activation of CD25⁺ NK cells upon stimulation with Semaphorin 5A,
- Figure 6 shows the presence of NKG2A and NKG2D receptors on NK cells upon stimulation with Semaphorin 5A,
- Figure 7 shows the upregulation of mRNA specific for Granzyme B in NK cells by Semaphorin 5A,
- Figure 8 shows the effect of Semaphorin 5A increasing the cytotoxicity ofNK cells against target cells,
- Figure 9 shows the effect of Semaphorin 5A on the proliferation of CD8⁺ T-cells, and
- Figure 10 shows the upregulation of Granzyme B in CD8⁺ T-cells by Semaphorin 5A.

In the figures, p<0.05 is indicated by a *, and p<0.01 is indicated by a **, which is both considered as statistically significant in the Mann-Withney U-test applied in the statistical analysis. In the figures, the same abbreviations denote the same samples or experiments, respectively.

For identifying a marker that is suitable for identifying a status of activity of the immune system indicating GvH disease, approximately 60 patients having received hematopoietic stem cell therapy using allogeneic stem cells were monitored for the GvH disease, i.e. the activation of the immune cells was monitored. In accordance with standard practice, this was done by a biopsy, because no validated diagnostic blood test is available that can determine the graft versus host disease, i.e. an activation of the immune system in response to antigen.

When searching for a biomarker that is indicative of the activation of the immune system or occurring in the GvH disease, it has been found that the level of Semaphorin 5A protein in peripheral blood is a reliable marker for the presence of an increased activity of the immune system, i.e. for the immune response of the graft versus host disease in the transplant patients.

It has been found that levels of Semaphorin 5A, preferably determined as Semaphorin 5A mRNA, an increase by a factor of at least 2, preferably by a factor of at least 4 to 5, preferably by a factor of at least 10, more preferably by a factor of about 15, and most preferably by a factor of about 20 or above in relation to an average concentration of Semaphorin 5A indicates an increased activity of the immune system, which is e. g. indicative of GvH disease in hematopoietic stem cell transplant recipients, or of an autoimmune disease. Accordingly, the diagnostic method preferably includes the step of normalizing the signal specifically detected for Semaphorin 5A to a constitutively expressed gene product, and determining the relation of the normalized signal for Semaphorin 5A to the signal for Semaphorin 5A which has been determined and normalized in a sample obtained from a healthy person. The resulting relation of the signal for Semaphorin 5A of the patient to the signal obtained in a healthy person can be edited, e.g. for subsequent medical evaluation.

Generally, Semaphorin 5A, or the mRNA encoding Semaphorin 5A, can be analysed by the method in a sample obtained from a patient, which sample is preferably a cell-containing sample, e.g. blood, and specifically peripheral blood mononuclear cells (PBMC).

Preferably, the concentrations of Semaphorin 5A are standardized by relating them to the signal determined for a constitutive sample component. For embodiments of the method for analysis determining the concentration of Semaphorin 5A by RT-PCR, i.e. by reverse transcription of RNA isolated from the sample to cDNA, followed by specific amplification of a fragment of the semaphorin 5A coding sequence by PCR, the β-actin encoding sequence can be the constitutive sample component, in relation to which the signal specific for Semaphorin 5A is normalized.

As an alternative to determining the mRNA by reverse transcription of RNA to produce cDNA, followed by PCR on the basis of the cDNA using primers specific for the semaphorin 5A coding sequence, a specific antibody reaction against Semaphorin 5A can be used. Polyclonal, preferably monoclonal antibody that is specifically directed against Semaphorin 5A can be produced by standard methods, e.g. by immunizing an experimental animal with Semaphorin 5A and collecting serum for polyclonal antibody, or using spleen cells from the immunized animal for generation of hybridoma and selecting a clone to produce a monoclonal antibody.

When analysing the activity of Semaphorin 5A (protein) on immune cells in *in vitro* assays, it has been found that the addition of Semaphorin 5A to the cultivation medium increases the cytotoxic activity of NK cells, the cytotoxic activity of CD8⁺ T-cells, and the proliferation of CD8⁺ T-cells, also in the absence of target cells, e.g. of allogeneic cells. Accordingly, in the second embodiment, the invention provides Semaphorin 5A in a pharmaceutical composition or as an ingredient in a medicament for use in the activation of the immune system, especially for activating the cytotoxic activity ofNK cells, for activating the cytotoxic activity of CD8⁺ T-cells, and for the proliferation of CD8⁺ T-cells. Preferably, the use of Semaphorin 5A is in the treatment of an insufficient immune system. The use of Semaphorin 5A as a medicament for the treatment of a low activity of the immune system, and Semaphorin 5A for use in the preparation of a pharmaceutical composition for use in the treatment of a low activity of the immune system, e.g. for increasing the activity of the immune system, preferably in patients suffering from congenital or aquired immune deficiences, infectious diseases or cancer.

More preferably, the Semaphorin 5A for use as a medicament is a combination of Semaphorin 5A with IL-2 and/or with IL-15 for use as a medicament, especially for activating the immune system.

### Example 1: Semaphorin 5A is specifically increased in GvH patients.

After written consent, as applicable, peripheral blood mononuclear cell samples were isolated from 60 patients (30-60 years old) who underwent allogeneic hematopoietic stem cell therapy. Prior to this therapy, similar conditioning regimens were applied. From these patients, 5 were suspected of GvH disease, 6 patients presented clinical signs of GvH disease at the time the analysis was made, or had been diagnosed with GvH disease up to two months before, and a further 6 patients had been diagnosed with clinical GvH disease at 6-2 months before the analysis was made. 43 of the patients never developed GvH disease or were free of GvH disease for more then 6 months. As a control, PBMCs from 10 healthy individuals was used.

Total RNA was isolated from PBMC samples, and reverse transcription was performed on RNA to produce complementary DNA (cDNA) using a high capacity cDNA reverse transcription kit (Applied Biosystems, Foster City, USA). For each reaction, 50 ng total RNA was used. Semaphorin 5A and, in parallel reactions, Semaphorin 3A and β-actin mRNA were determined using a two-step real time PCR (TaqMan Gene Expression Master Mix, Applied Biosystems, Foster City, USA) using amplification primers and FAM-labelled minor groove binding (MGB) Taqman probes.

For Semaphorin 5A, the primers corresponding to SEQ ID NO 1 (5'-CAACGGCGGCCACTATG -3') and to SEQ ID NO 2 (5'- CCAGGCGGGCTTTGC -3'), using MGB-probe of SEQ ID NO 3 (5'FAM- CAACGATTCCGATACACA 3') were used, for Semaphorin 3A primers having SEQ ID NO 4 (5'- TTCAGCAATGGAGCTTTCCA -3') and SEQ ID NO 5 (5'- CAACCCCAGCCGTTGAAC -3') with the MGB-probe according to SEQ ID NO 6 (5'-FAM- AAGCAGCAACAACTATATA -3') were used, and for β-actin primers according to SEQ ID NO 7 (5'- ATG ATG ATA TCG CCG CGC -3') and SEQ ID NO 8 (5'- GCC TTG CAC ATG CCG G -3'), and MGB-probe having SEQ ID NO 9 (5'-FAM- CGT CGT CGA CAA CGG -3') were used. For standardizing levels of Semaphorin 5A and of Semaphorin 3A mRNAs, respectively, the specific amount of β-actin-mRNA of parallel aliquots was used. Thermal cycling in a Step One plus real-time PCR system (Applied Biosystems) was 95°C for 10min, followed by 40 cycles at 95°C for 15s and 60°C for 1min. For analysis, the Step One software version 2.0 (Applied BioSystems) and a quantification-comparative method were used. Generally, patient data for Semaphorin 5A were normalized to the Semaphorin 5A signals of the healthy control group after normalizing each signal of Semaphorin 5A to that of β-actin.

The mRNA for human Semaphorin 5A is available under accession number NM_003966.2 from GenBank (SEQ ID NO 10). Preferably, the nucleotide sequence of Semaphorin 5A corresponds to SEQ ID NO 10, and the specific identification of Semaphorin 5A uses a nucleotide sequence according to SEQ ID NO 10, e.g. contained in a sample and used in the above described RT-PCR. Further, Semaphorin 5A protein according to this invention is preferably encoded by nucleotide sequence according SEQ ID NO 10, more preferably from nt 779 to nt 3934, and most preferably, Semaphorin 5A protein according to this invention has an amino acid sequence according to SEQ ID NO 11.

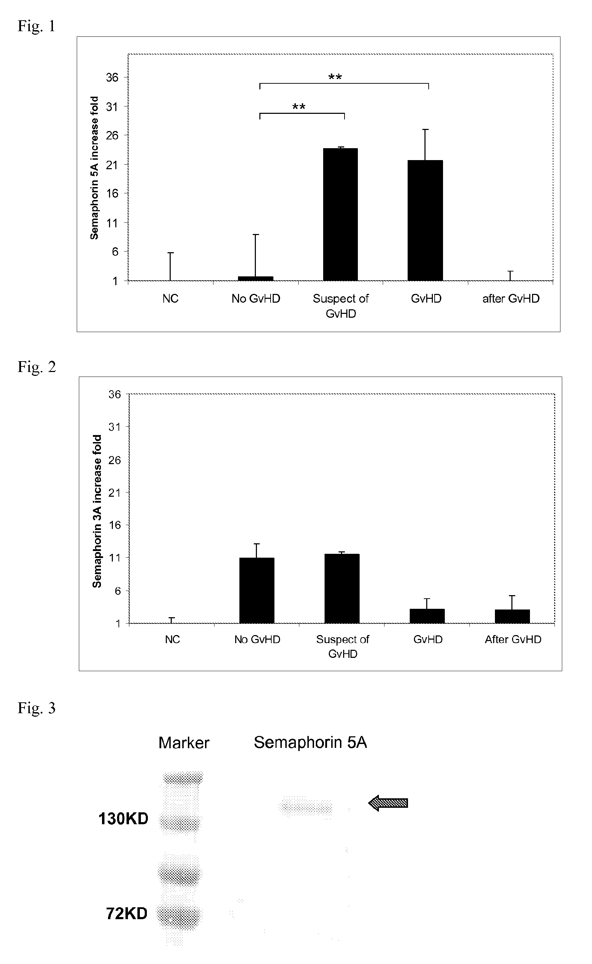

It was found that in comparison to the group without GvH disease (relative quantification (RQ):1.7±7.2), the patients suspected of GvH disease (RQ: 23.7±0.33; p<0.001) and patients with clinical diagnosis of GvH disease (RQ: 21.7±5.3; p<0.001) showed a significant increase of mRNA levels specific for Semaphorin 5A. After hematopoietic stem cell transplantation, no significant difference in the levels of Semaphorin 5A mRNA was observed in the group of patients after GvH disease (RQ: 1±1.7), when compared to the group without GvH disease. Results are shown in Figure 1, wherein mRNA levels of Semaphorin 5A are normalized to β-actin mRNA levels and the levels of Semaphorin 5A mRNA are normalized to the values of the healthy control group. The data show that the level of Semaphorin 5A in PBMCs is indicative of the increased activity of the immune system occurring in GvH disease. In detail, the group of patients which are suspected of GvH disease (Suspect of GvH) and the group of patients being diagnosed with GvH disease (GvHD) have an increase in the normalized mRNA levels of Semaphorin 5A by a factor of about 20 in comparison to the control group (NC), and in relation to the patients without GvH disease (No GvHD). After treatment of the patients suffering from GvH disease (after GvHD), i.e. by standard immunosuppressant therapy, levels of Semaphorin 5A were in the same range as those of healthy control and no GvHD-patients, demonstrating the diagnostic value of Semaphorin 5A levels for GvH disease.

The analysis of Semaphorin 3A in the same patient samples showed that Semaphorin 5A was unregulated in GvH disease-cases, whereas Semaphorin 3A was not. In detail, for Semaphorin 3A, no difference was measured between patients without GvH disease (No GvHD, RQ: 10.9±2.25) and patients suspected of GvH disease (Suspect of GvHD, RQ: 11.5± 0.4), and no differences were observed in patients diagnosed with GvH disease (GvHD, RQ: 3.2±1.59) and patients after GvH disease (After GvHD, RQ: 3.1±2.1), which results are shown in greater detail in Fig. 2.

### Example 2: Production of recombinant Semaphorin 5A

As an example for the manufacture of Semaphorin 5A, e.g. for use in the production of a pharmaceutical composition containing Semaphorin 5A for use in the treatment of a low activity of the immune system, the extracellular domain of human Semaphorin 5A was expressed in HEK cells. For ease of purification, the expression construct included a His-tag. Briefly, the plasmid pcDNA3.1 was provided with the coding sequence for the extracellular domain of human Semaphorin 5A with an added soluble His-tag, and transfected using lipofection (Lipofectamine 2000, Invitrogen) into the human embryonic kidney cell line HK293. The recombinant Semaphorin 5A protein with its added His-tag was isolated from cell culture supernatant by immobilized-metal affinity chromatography on HisTrapFF columns (GE Healthcare, Little Chalfont, Great Britain). Cell culture supernatants were pooled, adjusted to pH 8.0, and loaded onto the columns using a BioLogic DuoFlow System, Bio-Rad, Hercules, USA). Elution of Semaphorin 5A was by 20 mM sodium phosphate, 0.5 M sodium chlorate, 0.5 M imidazole, pH 7.4. Recombinant protein was quality-controlled by Western blotting and an ELISA using detection antibodies against the V5 epitope and the His-tag (available from Invitrogen). The result is shown in Fig. 3, with the arrow indicating Semaphorin 5A.

### Example 3: Production of an antibody having specificity against Semaphorin 5A

Polyclonal and monoclonal antibody specific for Semaphorin 5A was produced according to standard practice in experimental animals. In short, experimental mice were vaccinated with Semaphorin 5A as produced according to example 2, using at least 2 booster vaccinations. For polyclonal antibody, serum was collected and purified as generally known. The specificity could be determined against immobilized Semaphorin 5A by detecting bound antibody using a secondary antibody.

For monoclonal antibody, spleen cells of a mouse were used for the hybridoma technique, and from hybridoma cultures, a producer was screened according to the affinity of the supernatant to immobilized Semaphorin 5A.

### Example 4: Activation NK cells and of the cytotoxic activity of NK cells by Semaphorin 5A

Natural killer cells (NK cells) were isolated from peripheral blood of 8 healthy donors using magnetic assisted cell separation technology. Cells were cultured in the presence of IL-2 and IL-15, in the presence of Semaphorin 5A alone, or in a combination of Semaphorin 5A with IL-2 and IL-15. As a control, heat-denatured Semaphorin 5A was used. Cultivation was generally in standard cell culture medium under standard cultivation conditions.

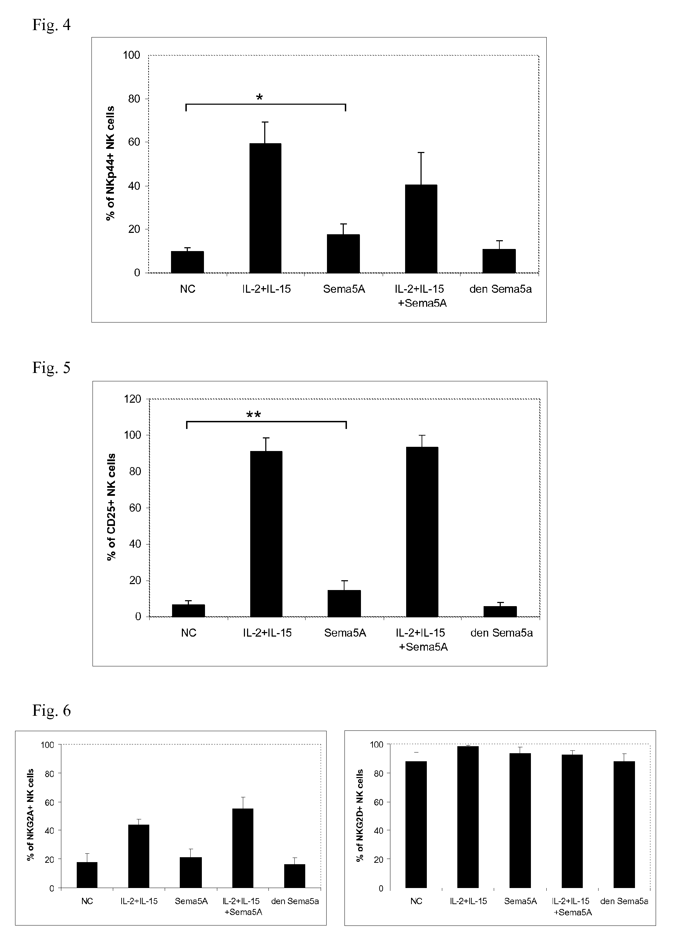

After 48 hours of cultivation, the following cell surface receptors were determined by flow cytometry specific for cell surface expression of KIR2DL1, KIR2DL2, NKp30, NKp44, NKp46, NKG2A, NKG2D, and CD25. Results are shown in Fig. 4 and demonstrate, that the exposure of the immune cells to Semaphorin 5A (Sema5A) lead to increase of the natural cytotoxic receptor NKp44, i.e. NKp44+ NK cells by approximately 12%. As expression of NKp44 is restricted to activated NK cells, this result demonstrates that Semaphorin 5A activates NK cells. This activation ofNK cells is even more pronounced for the combination of Semaphorin 5A with IL-2 and IL-15 (IL-2+ IL-15+Sema5A), whereas control cells (NC) without Semaphorin 5A and cells exposed to denatured Semaphorin 5A (den Sema5a) show lower activation.

In addition, exposure of the cultivated immune cells to Semaphorin 5A stimulated an increase of the frequency of CD25⁺ NK cells by up to 20%, as shown in Fig. 5. The stimulation is even more pronounced when Semaphorin 5A is used in combination with IL-2 and IL-15.

As shown in Fig. 6, the NKG2A+ (21.2%±5.7%) and NKG2D+ (93.8%±4%) NK cells increased significantly in the presence of Semaphorin 5A, when compared to NK cells that had been cultivated in the absence of Semaphorin 5A (NC), or NK cells that were incubated in the presence of Semaphorin 5A alone. No significant changes in the expression were observed for expression of NKp30, NKp46, KIR2DL1 or KIR2DL2 (data not shown).

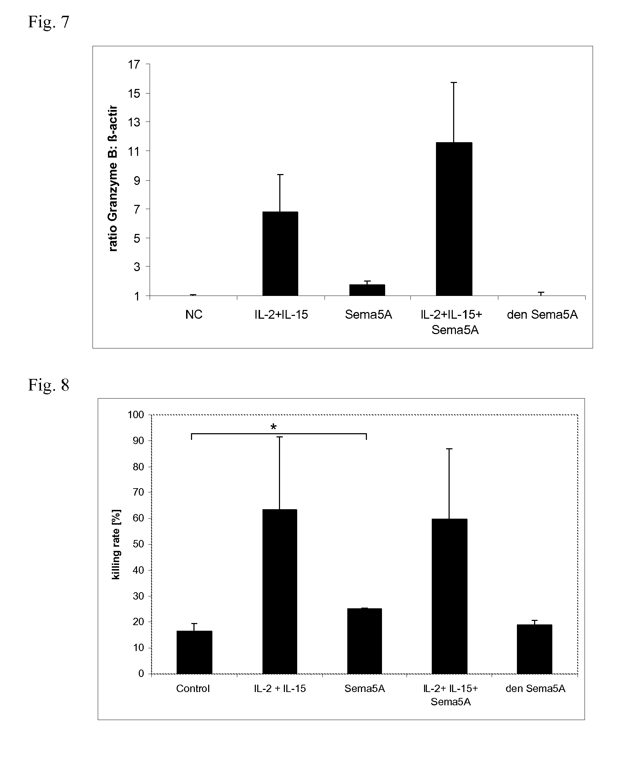

This data show that Semaphorin 5A, e.g. in the form of recombinant Semaphorin 5A, and preferably in the presence of additional IL-2 and/or IL-15, is an activator ofNK cell function. As shown in Figure 7, Semaphorin 5A stimulates NK cells to have increased levels of Granzyme B (1.72±0.31) in comparison to NK cells cultivated without Semaphorin 5A (1±0.05). mRNA levels of Granzyme B were evaluated by real-time PCR for determining its upregulation. The data shown are normalized to β-actin levels determined in aliquots.

An increased, e.g. synergistic effect for the upregulation of Granzyme B transcripts was observed for Semaphorin 5A in combination with both IL-2 and IL-15 (11.57±4.11), when compared to NK cells cultivated in IL-2 and IL-15 but without Semaphorin 5A (6.7±2.5). This clearly shows that Semaphorin 5A enhances the cytotoxic potential ofNK cells, even in the absence of target cells. As a control, non-simulated NK cells were used.

In an additional experiment, the NK cells previously incubated in the presence or absence of Semaphorin 5A, as described above, were used in cytotoxicity assays with K562 cells as targets. NK cells previously cultivated in the presence of Semaphorin 5A showed an increased lysing capacity of the target cells (25%±0.3%) in comparison to NK cells cultivated in the absence of Semaphorin 5A (16.5%±3%), as is also shown in Fig. 8.

### Example 5: Stimulation of proliferation of CD8⁺ T-cells, and increase of cytotoxicity of CD8⁺ T-cells by Semaphorin 5A.

CD8⁺ T-cells were isolated from the peripheral blood of 4 healthy donors using magnetic assisted cell separation. Prior to the proliferation assays, CD8⁺ T-cells were labelled with 5,6-carboxy-succinimidyl-fluorescein-ester (CFSE), and cultured in the presence of IL-2 (50U), Semaphorin 5A (10µg) alone, or in a combination of Semaphorin 5A (10µg) and IL-2 (50U) for 5 days under standard cell cultivation conditions. In comparison to CD8⁺ T-cells cultivated without added stimulant (Control), cultures supplemented with IL-2 showed a 4% increase in the proliferation rate for CD8⁺ T-cells. A strong increase of the proliferation rate by about 10% was observed in the culture of CD8⁺ T-cells in the presence of Semaphorin 5A. Essentially no synergistic effect onto the proliferation rate was detected for the combination of IL-2 and Semaphorin 5A in this culture of CD8⁺ T-cells.

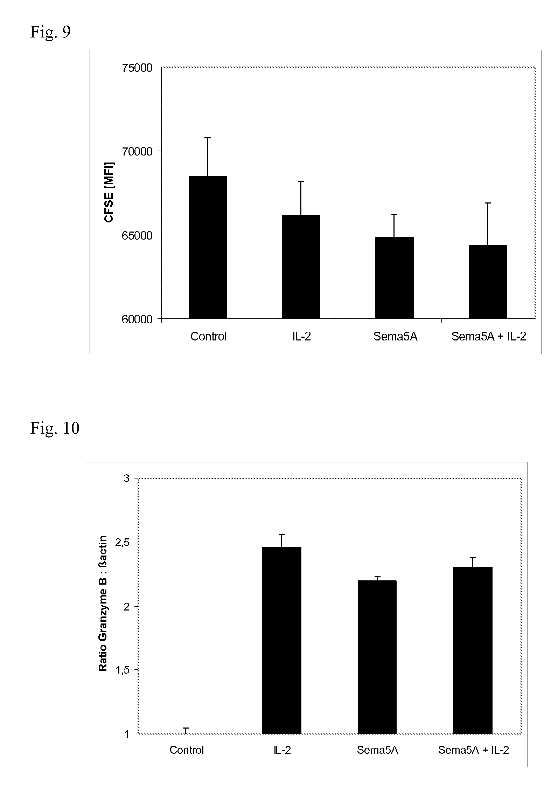

For an evaluation of the cytotoxic potential of CD8⁺ T-cells, total RNA was isolated from the individual cultures, and the level of Granzyme B transcripts were measured by real-time PCR. Data are shown in Fig. 10. Presence of IL-2 in the cultivation of CD8⁺ T-cells caused a 2.4±0.1 -fold increase in levels of Granzyme B. Presence of Semaphorin 5A alone, i.e. without additional IL-2, also induces an upregulation of Granzyme B-transcripts by a factor of 2.2±0.1. The combination added IL-2 and Semaphorin 5A lead to an increase of levels of Granzyme B by a factor of 2.3±0.07. These data demonstrate that it is Semaphorin 5A alone that is capable of inducing cell proliferation and an increased cytotoxicity of CD8⁺ T-cells..

## Claims

1. Method for analysis of the state of activation of the immune system of a patient, **characterized by** the determination of the concentration of Semaphorin 5A in a sample of the patient.

2. Method according to claim 1, **characterized in that** the concentration of Semaphorin 5A is determined by detecting the mRNA encoding Semaphorin 5A, or by detecting the reaction of an antibody that is specific for Semaphorin 5A protein.

3. Method according to claim 1, **characterized in that** the concentration of Semaphorin 5A is normalized to a signal determined for a constitutively expressed constituent of the sample.

4. Kit of components for use in a method according to one of the preceding claims, comprising a pair of oligonucleotides specifically hybridising to a nucleotide sequence containing at least a fraction of the nucleotide sequence encoding Semaphorin 5A.

5. Kit according to claim 4, **characterized in that** it further comprises a pair of oligonucleotides specifically hybridising to a nucleotide sequence encoding at least a fraction of a constitutively expressed mammalian protein.

6. Kit of components for use in a method according to one of claims 1 to 3, comprising an antibody which is specific for Semaphorin 5A.

7. Kit according to claim 6, **characterized in that** it further comprises an antibody which is specific for a constitutively expressed mammalian protein.

8. Semaphorin 5A protein for use as a medicament.

9. Semaphorin 5A for use according to claim 8, **characterized in that** the medicament is used for activating the immune system.

10. Semaphorin 5A for use according to claim 8 or 9, **characterized in that** Semaphorin 5A has an amino acid sequence according to SEQ ID NO 11.

11. Semaphorin 5A for use according to one of claims 8 or 9, **characterized in that** Semaphorin 5A is in combination with IL-2 and/or IL-15.

12. Use of Semaphorin 5A in the production of a pharmaceutical composition.

13. Use according to claim 11, **characterized in that** the pharmaceutical composition is prepared for use in the activation of the immune system of a patient.
